# EUROPÄISCHE PATENTANMELDUNG

(11) **EP 2 239 676 A1**
(43) Veröffentlichungstag der Anmeldung: **13.10.2010**
(21) Anmeldenummer: 10157116.4
(22) Anmeldetag: 19.03.2010
(51) Int. Cl.: G06F 19/00

(54) **Verfahren und Anordnung zur Vorhersage mindestens eines Systemereignisses sowie ein entsprechendes Computerprogramm und ein entsprechendes computerlesbares Speichermedium**

(30) Priorität: 09.04.2009 DE 102009002307; 18.01.2010 DE 102010000103; 07.08.2009 US 232012 P
(71) Anmelder: Biotronik CRM Patent AG, 6341 Baar (CH)
(72) Erfinder: Ebert, Manuel, 91083, Baiserdorf (DE); Bast, Daniel, 63785, Obernburg (DE); Krämer, Thomas, 90425, Nürnberg (DE); Berdyshev, Sergey, 91083, Baiersdorf (DE); Meyer, Wolfgang, 91052, Erlangen (DE)
(74) Vertreter: Lindner-Vogt, Karin L.

(57) **Zusammenfassung**

Gegenstand der Erfindung ist ein Verfahren und eine Anordnung zur Vorhersage mindestens eines Systemereignisses sowie ein entsprechendes Computerprogramm und ein entsprechendes computerlesbares Speichermedium, wobei sich das Ereignis aufgrund von Trends in Observablen über einen gewissen Zeitraum vor Eintreten des Ereignisses vorhersehen lässt. Die Erfindung ist insbesondere für ein patientenindividuelles Monitoring pathophysiologischer Veränderungen einsetzbar, aber auch in geophysikalischen oder abstrakten Einheiten wie volks- oder betriebswirtschaftlichen Systemen, bei denen eine Abweichung eines definierten Normalzustandes vorhergesagt wird.

Hierfür ist vorgesehen, zur Vorhersage mindestens eines Systemereignisses zumindest folgende Schritte auszuführen:
- Erfassung von Messwerten sⱼ mindestens eines Primärparameters Sⱼ innerhalb mindestens eines Zeitfensters vorgebbarer Länge,
- für zumindest einen Teil der erfassten Primärparameter Sⱼ Ermittlung einer Trendwahrscheinlichkeit pⱼ für eine vorgebbare Anzahl von Zeitfenster,
- für zumindest einen Teil der erfassten Primärparameter Sⱼ Ermittlung eines Wertes für die Relevanz der Zeitfenster für die Vorhersage des mindestens einen Systemereignisses,
- für zumindest einen Teil der erfassten Primärparameter Sⱼ und für zumindest einen Teil der Zeitfenster Konstruktion mindestens eines Merkmals M zur Beschreibung des Systems durch Auswertung der Trendwahrscheinlichkeit(en) pⱼ und des Wertes für die Relevanz der Zeitfenster,
- Klassifikation des mindestens einen Merkmals M und
- Signalisierung eines Systemereignisses und/oder Einleitung systemereignisbezogener Maßnahmen in Abhängigkeit der Ergebnisse der Klassifikation.

## Beschreibung

### Einführung

Gegenstand der Erfindung ist ein Verfahren und eine Anordnung zur Vorhersage mindestens eines Systemereignisses sowie ein entsprechendes Computerprogramm und ein entsprechendes computerlesbares Speichermedium, wobei sich das Ereignis aufgrund von Trends in Observablen über einen gewissen Zeitraum vor Eintreten des Ereignisses vorhersehen lässt. Die Erfindung ist insbesondere für ein patientenindividuelles Monitoring pathophysiologischer Veränderungen einsetzbar. Das Verfahren ist bei linearen und nichtlinearen Verläufen sowie bei Sprüngen der Observablen anwendbar. Ein Ereignis kann z.B. der Ausfall eines Systems sein, weil sich das abnormale Verhalten irgendeiner Komponente in Unregelmäßigkeiten einer oder mehrerer Observablen niederschlägt oder die Früherkennung bzw. präakute Vorhersage z.B. eines bestimmten kritischen Zustandes eines Patienten. Die Systeme an sich können auch geophysikalische oder abstrakte Einheiten wie volks- oder betriebswirtschaftliche Systeme repräsentieren, bei denen eine Abweichung eines definierten Normalzustandes vorhergesagt wird.

Bei komplexen Vorgängen oder Systemen werden Ereignisse durch eine Vielzahl von Faktoren ausgelöst. Diese Faktoren können ein heterogenes Auftreten aufweisen, so dass auch verschiedenartiges Verhalten von Faktoren zum selben Ereignis führen kann. Vorhersagen von Ereignissen, wie beispielsweise einer sog. Dekompensation bei Herzpatienten, sind daher bisher nur sehr ungenau möglich.

Es ist daher Aufgabe der vorliegenden Erfindung, ein Verfahren und eine Anordnung zur Vorhersage mindestens eines Systemereignisses sowie ein entsprechendes Computerprogramm und ein entsprechendes computerlesbares Speichermedium für die Vorhersage eines Ereignisses bereitzustellen, welche die genannten Nachteile vermeiden und insbesondere robust gegen Heterogenität von Signalverläufen vor bestimmten Ereignissen sind.

Erfindungsgemäß ist bei dem Verfahren zur Vorhersage von Systemereignissen vorgesehen, dass Messwerte sⱼ mindestens eines Primärparameters Sⱼ innerhalb mindestens einem Zeitfenster erfasst werden. Die Zeitfenster haben eine vorgebbare Länge *L* ∈ *N*, die zwischen einer kleinsten Länge *L*ₘᵢₙ ∈ *N* und einer größten Länge *L*ₘₐₓ ∈ *N* variiert, und die zu vorgebbaren Zeitpunkten *H* ∈ *N* beginnen. Die Variable H beschreibt die Vorlaufzeit zwischen dem Zeitpunkt der Prädiktion und dem Eintreten des vorhergesagten Ereignisses, also z.B. die Zeit bis zum tatsächlichen oder vermuteten Auftreten des bevorstehenden Systemereignisses und läuft von *H*ₘᵢₙ ∈ *N* bis *H*ₘₐₓ ∈ *N*. Für zumindest einen Teil dieser Zeitfenster und für zumindest einen Teil der Primärparameter Sⱼ wird die Wahrscheinlichkeit für das Vorliegen eines Trends bestimmt. Des Weiteren wird für zumindest einen Teil der Zeitfenster und für zumindest einen Teil der Primärparameter Sⱼ ein Wert für die Relevanz (Relevanzwert) dieses Fensters für die Vorhersage bestimmt. Vorzugsweise wird dabei zumindest die Trennschärfe des Zeitfensters für den Primärparameter Sⱼ ermittelt. Für zumindest einen Teil der erfassten Primärparameter Sⱼ und für zumindest einen Teil der Zeitfenster wird anschließend mindestens ein Merkmal konstruiert, wobei bei der Konstruktion des mindestens einen Merkmals zumindest ein Teil der Trendwahrscheinlichkeiten pⱼ und der Relevanzwerte berücksichtigt werden. Diese Berücksichtigung kann darin bestehen, dass Trendwahrscheinlichkeiten pⱼ und Relevanzwerte durch geeignete arithmetische Operationen miteinander verknüpft werden. Beispielsweise kann eine Gewichtung der Trendwahrscheinlichkeiten pⱼ in Abhängigkeit der Relevanzwerte und/oder eine Summation der (gewichteten) Trendwahrscheinlichkeiten pⱼ vorgesehen werden. In den Relevanzwert fließt in einer bevorzugten Ausführungsform die Trennschärfe des Zeitfensters für den Primärparameter Sⱼ ein. Auf das Merkmal wird ein Klassifikationsverfahren angewendet, welches einen Indikator für das Einteten oder Ausbleiben eines Systemereignisses liefert. In Abhängigkeit diese Indikators erfolgt eine Signalisierung des Systemereignisses und/oder es werden systemereignisbezogene Maßnahmen initiiert.

Bei der Erfindung wird ein Multiparameteransatz verwendet, bei dem für einzelne Primärparameter Sⱼ Merkmale, ausgehend von einer Trendbetrachtung, bestimmt werden, die dann in ein Klassifikationsverfahren einfließen. Jedes Merkmal basiert jeweils auf mindestens einem Primärparameter Sⱼ. In einer bevorzugten Ausführungsform werden alle Trendwahrscheinlichkeiten pⱼ zur Identifikation von Bereichen mit größter Trennschärfe herangezogen. Daraus leiten sich dann die relevanten Fensterlängen und -positionen ab. In diesen Fenstern werden vorzugsweise alle Wahrscheinlichkeiten kombiniert (und nicht nur diejenigen, die größer sind als ein Schwellenwert). Diese Kombination stellt dann ein Merkmal für ein Klassifikationsverfahren dar, das wiederum aus jedem Primärparameter Sⱼ gebildet werden kann.

Beim Einsatz der Erfindung wird also der Signalverlauf eines Systems in einem beschränkten Zeitraum vor einem zu detektierenden Ereignis auf eine bestimmte Form der Veränderlichkeit überprüft, indem die Wahrscheinlichkeit für das Vorliegen eines Trends von Zeitfenstern, die zu gegebenen Zeiten vor dem Prädiktionszeitpunkt beginnen und gegebene Längen haben, berechnet wird. Diese Wahrscheinlichkeiten ausgewählter Zeitfenster werden kombiniert. Diese Kombination wird vorzugsweise für alle Messgrößen berechnet. Im Anschluss wird gegebenenfalls eine geeignete Zusammenfassung der Kombinationen für die Messgrößen vorgenommen und Merkmale werden konstruiert. Das hierauf angewendete Klassifikationsverfahren wird entweder mit Hilfe von Daten mit bekannten Eigenschaften optimiert oder auf unbekannte Daten angewendet und das Ergebnis des Verfahrens unter Zuhilfenahme einer maschinellen oder manuellen Entscheidungsgrenze ausgewertet.

Unter Zuhilfenahme eines solchen geeigneten Klassifikationsverfahrens wird ein Indikator geliefert, anhand dessen auf das Eintreten (oder Ausbleiben) eines Ereignisses geschlossen werden kann. Ein solcher Indikator zeigt dann ein bevorstehendes Ereignis an. Bei solchen Ereignissen kann es sich beispielsweise um ein kritisches Systemereignis handeln. Bei solchen kritischen Systemereignissen kann es sich beispielsweise um Arrhythmien, Lungenembolien, Schlaganfälle, Myokardinfarkte, Angina Pectoris, Syncope, transitorische ischämische Attacken und/oder akute periphere arteriosklerotische Gefäßerkrankungen handeln.

Aus dem Ergebnis werden verschiedene Vorgehensweisen wie z. B. die Auslösung eines Alarms abgeleitet, der das Fehlverhalten eines Systems anzeigt, Abweichungen vom Normalverhalten signalisiert, automatisierte Wartungs- oder Substitutionsprozesse in Gang setzt, eine Krankheitsverschlechterung dem Arzt oder Patienten meldet, eine unterstützende Therapie, z.B. einen Schock oder die Änderung des Pacing-Schemas (Stimulationsschemas) einleitet oder die Einleitung ähnlicher zeitnaher Maßnahmen erlaubt, um Schäden am System, den Ausfall des Systems oder den Tod des Patienten zu vermeiden.

Die Erfindung stellt ein Verfahren zur Vorhersage von Systemereignissen in der Zukunft bereit. Die Vorhersagen erfolgen anhand von Primärparametern, die in einem beschränktem Zeitraum vor dem Vorhersagezeitpunkt erfasst wurden, wobei aus den Primärparametern, die das System in einem bestimmten Zeitbereich charakterisieren, Merkmale gebildet werden, die die Veränderung des Systems widerspiegeln. Der Vorhersagezeitraum kann nach vom und hinten begrenzt sein und muss nicht notwendigerweise am Vorhersagezeitpunkt beginnen.

Hierfür wird innerhalb des Beobachtungszeitraums mindestens ein Zeitfenster der Länge *L* betrachtet. Innerhalb des Zeitfensters werden Messwerte *sⱼ* mindestens eines Primärparameters *Sⱼ* erfasst. Die Messwerte zu den Messpunkten der Primärparameter vor dem Prädiktionszeitpunkt werden in zeitlicher Reihenfolge verwendet.

Für zumindest einen Teil der Zeitfenster wird jeweils eine Kombination der Trendwahrscheinlichkeiten für zumindest einen Teil des mindestens einen Primärparameters *Sⱼ* bestimmt. Es können auf- und absteigende Trends gemeinsam oder getrennt betrachtet werden.

Erfindungsgemäß ist daher vorgesehen, dass auf Basis der Trendwahrscheinlichkeiten von Zeitfenstern eine Signalisierung eines Systemereignisses erfolgt und/oder eine oder mehrere systemereignisbezogene Maßnahmen initiiert werden. Dies kann beispielsweise dadurch erreicht werden, dass die Kombination der Trendwahrscheinlichkeiten von Zeitfenstern in einem Klassifikationsverfahren ausgewertet werden und anhand der Klassifikation die Vorhersage des mindestens einen Systemereignisses erfolgt. Bei solchen Maßnahmen kann es sich beispielsweise um systemstabilisierende Maßnahmen handeln, wobei systemstabilisierende Maßnahmen z.B. Medikamentierungsänderungen und/oder andere therapeutische Unterstützungsmaßnahmen, automatisierte Wartungs- und/oder Substitutionsprozesse umfassen.

Eine bevorzugte Ausführungsform sieht vor, dass die Klassifikationsverfahren unter Verwendung von klassifizierten Ereignisdaten und/oder von Kontrolldaten optimiert werden.

Eine andere bevorzugte Ausführungsform sieht vor, dass die Bestimmung des Trends die Prüfung einer Hypothese auf das Vorliegen eines Trends umfasst. Vorzugsweise wird dabei der Test auf Trend nach Mann durchgeführt. Hier sind auch andere Verfahren zum Test auf Trend anwendbar, der Test auf Mann ist jedoch im Vergleich zu vielen anderen Verfahren robuster gegenüber Ausreißern.

Eine weitere bevorzugte Ausführungsform sieht vor, dass für die Bestimmung des Trends für verschiedene Primärparameter verschiedene Verfahren zum Test auf Trend zum Einsatz kommen, abhängig von der Charakteristik der Primärparameter.

Eine andere bevorzugte Ausführungsform sieht vor, dass man neben der Bestimmung des Trends auch die dazugehörige Trendstärke betrachtet.

Vorzugsweise werden die Messwerte (= Messsignale) *sⱼ* des mindestens einen Primärparameters *Sⱼ* in bestimmten Zeitintervallen erfasst. Die Einheiten der Zeitintervalle können Sekunden, Minuten, Stunden, Tage, Wochen, Monate oder Jahre sein, wobei die Zeitintervalle für die verschiedenen Primärparameter verschieden sein können.

Mit der Erfindung können beispielsweise heterogene Signalverläufe ausgewertet werden. Die Signalverläufe können dabei linear oder nichtlinear sein oder große Sprünge aufweisen.

Ein weiterer Vorteil der Erfindung besteht darin, dass das Verfahren robust gegenüber Datenlücken ist.

### Als Primärparameter kommen beispielsweise aber nicht ausschließlich folgende in Frage:

Alle Primärparameter, die die Herzrate des Patienten beinhalten, wie z.B. die Herzrate über einen vordefinierten Zeitraum, die Herzrate während einer definierten Ruhephase, die Variabilität der Herzrate und ähnliche. Alle Primärparameter, die Impedanzen im Patienten bestimmen, sei es durch intrakardiale (bipolare und multipolare), intrathorakale Messungen oder durch Bestimmung mit externen Sensoren. Alle Primärparameter, die die Aktivität eines Patienten auf irgendeine Weise bestimmen. Alle Primärparameter, in denen der Anteil links- oder rechtsventrikulärer stimulierter, wahrgenommener oder anderer Events einfließt. Alle Primärparameter, die implantatabhängige Signale aufzeichnen. Alle Primärparameter, die Extrasystolen unabhängig ihres Ursprungsorts bestimmen. Alle Primärparameter, die die Hämodynamik eines Patienten oder sonstige Elastizitäten, Drücke, Volumina oder Abstände erfassen. Alle Primärparameter, die außerhalb eines Implantats bestimmt werden, wie Größen, die durch aus Funk angebundenen Sensoren gewonnen werden oder Größen von Geräten, die Daten außerhalb des Körpers aufzeichnen und diese telemetrisch der Auswertungseinheit übermitteln. Alle biomedizinischen Primärparameter wie Stimulationsschwellen, Elektrodenkonfigurationen, Sensorverstärkungen oder Versatzwerte. Alle Primärparameter wie Blutzuckerspiegel, andere Biomarker oder ähnliche Größen. Alle Primärparameter, die biometrische Informationen über den Patienten bestimmen. Alle Primärparameter, die zusätzliche Informationen über eine Medikamentierung aufzeichnen. Alle Primärparameter die aus elektrophysiologischen oder biochemischen Verfahren bestimmt werden. Alle Primärparameter, die Signale aus bildgebenden, akustischen oder mechanischen Verfahren erfassen. Alle oben genannten Primärparameter, die zuvor normiert und/oder skaliert wurden. Alle möglichen Kombinationen mehrerer der oben genannten Primärparameter.

Eine Anordnung nach der Erfindung weist mindestens einen Chip und/oder Prozessor auf und ist derart eingerichtet, dass ein Verfahren zur Vorhersage mindestens eines Systemereignisses ausführbar ist, wobei das Verfahren zumindest folgende Schritte umfasst:
- Erfassung von Messwerten sⱼ mindestens eines Primärparameters Sⱼ innerhalb mindestens eines Zeitfensters vorgebbarer Länge,
- für zumindest einen Teil der erfassten Primärparameter Sⱼ Ermittlung einer Trendwahrscheinlichkeit pⱼ für eine vorgebbare Anzahl von Zeitfenstern,
- für zumindest einen Teil der erfassten Primärparameter Sⱼ Ermittlung eines Wertes für die Relevanz der Zeitfenster für die Vorhersage des mindestens einen Systemereignisses,
- für zumindest einen Teil der erfassten Primärparameter Sⱼ und für zumindest einen Teil der Zeitfenster Konstruktion mindestens eines Merkmals M zur Beschreibung des Systems durch Auswertung der Trendwahrscheinlichkeit(en) pⱼ und des Wertes für die Relevanz der Zeitfenster,
- Klassifikation des mindestens einen Merkmals M und
- Signalisierung eines Systemereignisses und/oder Einleitung systemereignisbezogener Maßnahmen in Abhängigkeit der Ergebnisse der Klassifikation.

Ein Computerprogramm zum Vorhersagen von Systemereignissen ermöglicht es einer Datenverarbeitungseinrichtung, nachdem es in den Speicher der Datenverarbeitungseinrichtung geladen worden ist, ein Verfahren zur Vorhersage mindestens eines Systemereignisses durchzuführen, wobei das Verfahren zumindest folgende Schritte umfasst:
- Erfassung von Messwerten sⱼ mindestens eines Primärparameters Sⱼ innerhalb mindestens eines Zeitfensters vorgebbarer Länge,
- für zumindest einen Teil der erfassten Primärparameter Sⱼ Ermittlung einer Trendwahrscheinlichkeit pⱼ für eine vorgebbare Anzahl von Zeitfenstern,
- für zumindest einen Teil der erfassten Primärparameter Sⱼ Ermittlung eines Wertes für die Relevanz der Zeitfenster für die Vorhersage des mindestens einen Systemereignisses,
- für zumindest einen Teil der erfassten Primärparameter Sⱼ und für zumindest einen Teil der Zeitfenster Konstruktion mindestens eines Merkmals M zur Beschreibung des Systems durch Auswertung der Trendwahrscheinlichkeit(en) pⱼ und des Wertes für die Relevanz der Zeitfenster,
- Klassifikation des mindestens einen Merkmals M und
- Signalisierung eines Systemereignisses und/oder Einleitung systemereignisbezogener Maßnahmen in Abhängigkeit der Ergebnisse der Klassifikation.

In einer weiteren bevorzugten Ausführungsform der Erfindung ist vorgesehen, dass das erfindungsgemäße Computerprogramm modular aufgebaut ist, wobei einzelne Module auf verschiedenen Datenverarbeitungseinrichtungen installiert sind.

Vorteilhafte Ausführungsformen sehen zusätzlich Computerprogramme vor, durch welche weitere in der Beschreibung angegebene Verfahrensschritte oder Verfahrensabläufe ausgeführt werden können.

Solche Computerprogramme können beispielsweise (gegen Gebühr oder unentgeltlich, frei zugänglich oder passwortgeschützt) downloadbar in einem Daten- oder Kommunikationsnetz bereitgestellt werden. Die so bereitgestellten Computerprogramme können dann durch ein Verfahren nutzbar gemacht werden, bei dem ein Computerprogramm nach Anspruch 13 aus einem elektronischen Datennetz, wie beispielsweise aus dem Internet, auf eine an das Datennetz angeschlossene Datenverarbeitungseinrichtung heruntergeladen wird.

Um das erfindungsgemäße Verfahren zum Vorhersagen von Systemereignissen durchzuführen, ist vorgesehen, ein computerlesbares Speichermedium einzusetzen, auf dem ein Programm gespeichert ist, das es einer Datenverarbeitungseinrichtung ermöglicht, nachdem es in den Speicher der Datenverarbeitungseinrichtung geladen worden ist, ein Verfahren zur Vorhersage mindestens eines Systemereignisses durchzuführen, wobei das Verfahren zumindest folgende Schritte umfasst:
- Erfassung von Messwerten sⱼ mindestens eines Primärparameters Sⱼ innerhalb mindestens eines Zeitfensters vorgebbarer Länge,
- für zumindest einen Teil der erfassten Primärparameter Sⱼ Ermittlung einer Trendwahrscheinlichkeit pⱼ für eine vorgebbare Anzahl von Zeitfenstern,
- für zumindest einen Teil der erfassten Primärparameter Sⱼ Ermittlung eines Wertes für die Relevanz der Zeitfenster für die Vorhersage des mindestens einen Systemereignisses,
- für zumindest einen Teil der erfassten Primärparameter Sⱼ und für zumindest einen Teil der Zeitfenster Konstruktion mindestens eines Merkmals M zur Beschreibung des Systems durch Auswertung der Trendwahrscheinlichkeit(en) pⱼ und des Wertes für die Relevanz der Zeitfenster,
- Klassifikation des mindestens einen Merkmals M und
- Signalisierung eines Systemereignisses und/oder Einleitung systemereignisbezogener Maßnahmen in Abhängigkeit der Ergebnisse der Klassifikation.

Nachfolgend sollen nähere Einzelheiten von vorteilhaften Ausführungsformen der Erfindung erläutert werden:
- Figur 1: Beschreibung der Parameter Lₘₘ, Lₘₐₓ, Hₘᵢₙ und Hₘₐₓ;
- Figur 2a: Trendwahrscheinlichkeit für einen Ereignispatienten in Abhängigkeit von L und H;
- Figur 2b: Trendwahrscheinlichkeit für einen Kontrollpatienten in Abhängigkeit von L und H;
- Figur 3: Trennstärke eines Primärparameters für ein Patientenkollektiv;
- Figur 4: Nicht zusammenhängendes Gebiet hoher Trennstärken bei einem Primärparameter;
- Figur 5: Nassi-Shneiderman-Diagramm für die Berechnung der Trendwahrscheinlichkeiten;

### Verfahren

### Grundlegende Berechnung (Merkmalskonstruktion):

Im ersten Schritt werden die einlaufenden Messsignale *sⱼ* der Messgröße *Sⱼ* einer Filterung unterzogen. Die Messgröße *Sⱼ* mit *j*=1,...,*N^{S}* geht im Folgenden als sog. Primärparameter in die Merkmalskonstruktion ein. Die Anzahl der Primärparameter wird mit *N^{S}* bezeichnet (hochgestellte Zeichen und Ziffern sind Indices, wenn nicht anders vermerkt). Im ersten Schritt wird u.a. geprüft, ob die Werte der Messgrößen in einem gültigen Wertebereich liegen.

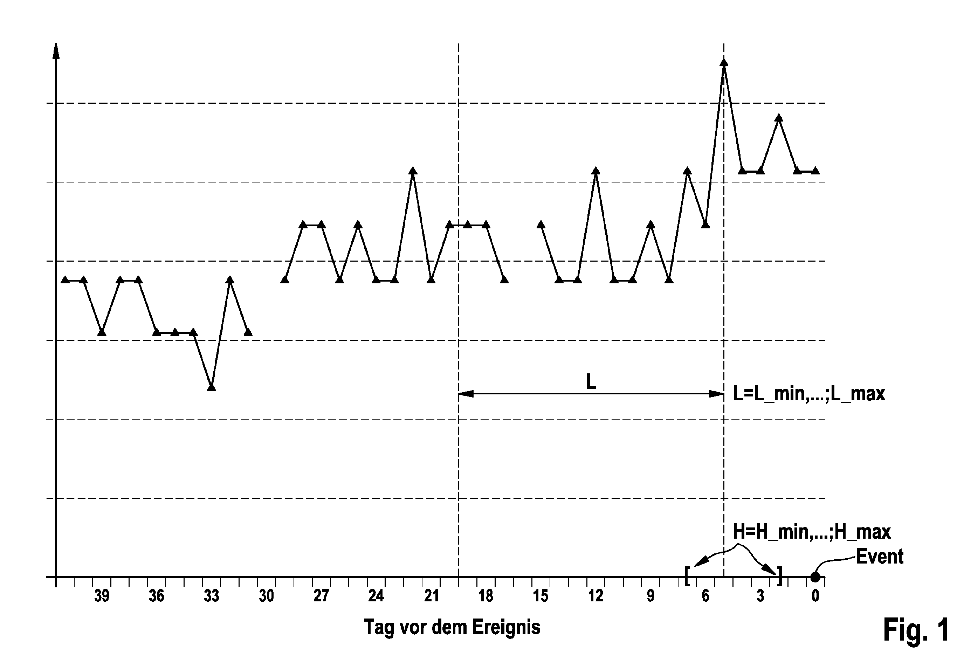

Die Zeitreihe der Messsignale ist prinzipiell von zwei Parametern abhängig, deren Bedeutung in Figur 1 skizziert ist. Die Variable *L* ∈ *N* stellt dabei ein Beobachtungsfenster zwischen den Längen *L*ₘᵢₙ ∈ *N* und *L*ₘₐₓ ∈ *N* dar, und in dessen Verlauf wird die Veränderlichkeit eines Messsignals geprüft. Die Variable *H* ∈ *N* beschreibt die Vorlaufzeit zwischen dem Zeitpunkt der Prädiktion und dem Eintreten des vorhergesagten Ereignisses, also z.B. die Zeit bis zum tatsächlichen oder vermuteten Auftreten des bevorstehenden Dekompensationsprozesses und läuft von *H*ₘᵢₙ ∈ *N* bis *H*ₘₐₓ ∈ *N*. Das Fenster sollte anschließend mit einem geeigneten Kriterium auf Gültigkeit überprüft werden. Eine Möglichkeit wäre die Forderung nach mind. 50% validen Messsignalwerten, die genaue Wahl hängt jedoch immer von der vorliegenden Problemstellung und/oder dem Charakter der Eingangssignale ab.

Für gültige Fenster wird z.B. aber nicht ausschließlich der Test auf Trend nach Mann verwendet. Hierbei wird für jedes Fenster die Größe $C = {\sum }_{k = 1}^{n - 1} {\sum }_{l = k + 1}^{n} sign ⁢ \left({y}_{l}-{y}_{k}\right)$
berechnet, wobei $sign ⁢ \left({y}_{l}-{y}_{k}\right) = { \begin{matrix}1 , & für {y}_{l} - {y}_{k} > 0 \\ 0 , & für {y}_{l} - {y}_{k} = 0 \\ - 1 , & für {y}_{l} - {y}_{k} < 0\end{matrix}$
die Signumsfunktion bezeichnet.

Die Hypothese eines negativen Trends wird bestätigt, falls $C < - {K}_{n , p} ,$
die Hypothese eines positiven Trends, falls $C > {K}_{n , p} .$
*K_{n,p}* ist hierbei das *p*-Quantil der Kendall'schen *K*-Statistik. Das Trendergebnis wird für jedes Parametertupel (*L*, *H*) einzeln berechnet.

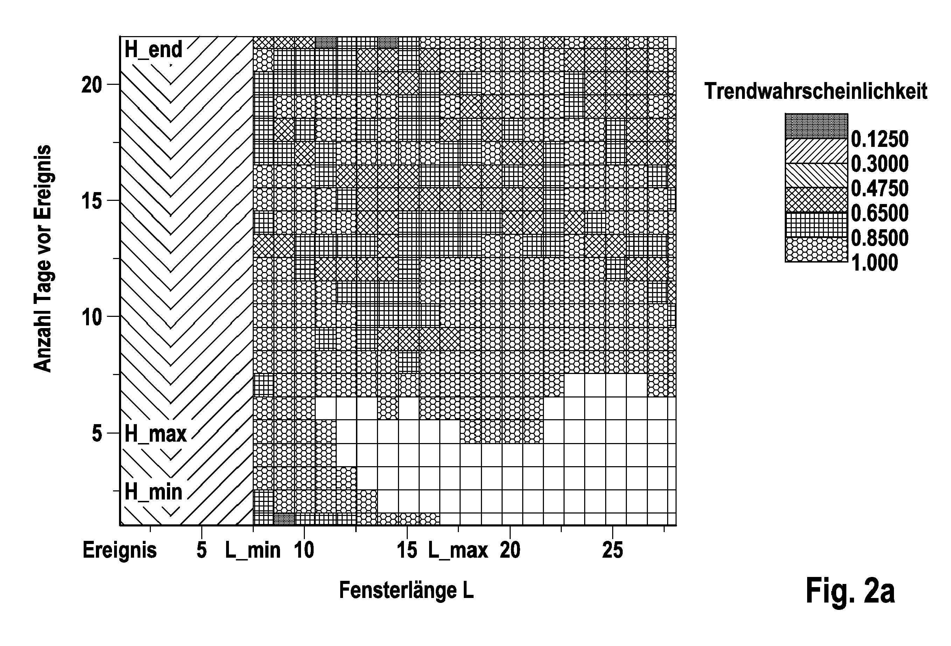

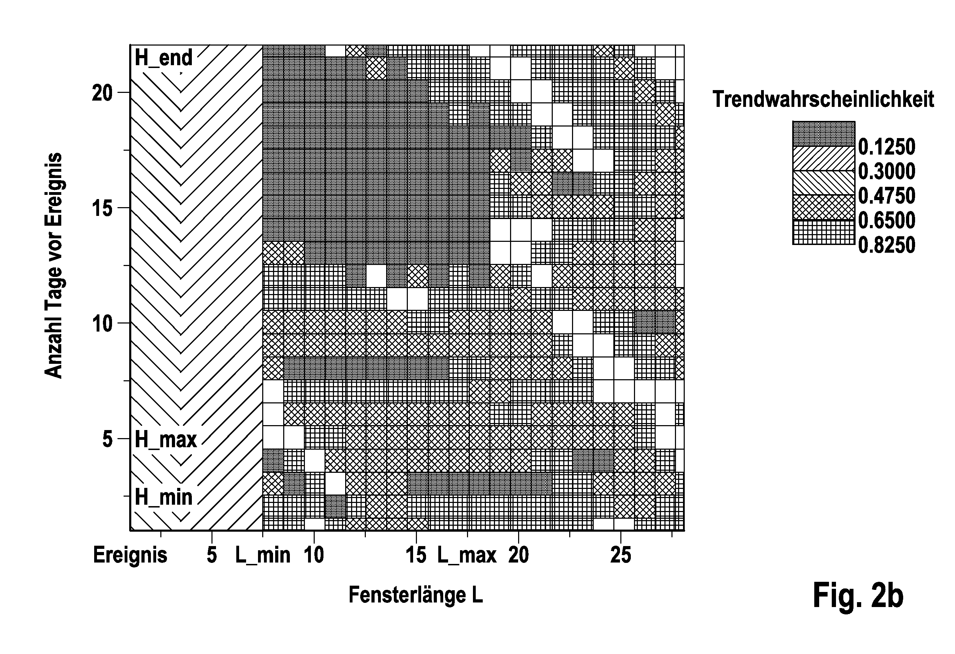

Für das weitere Vorgehen empfiehlt es sich, die jeweilige Trendwahrscheinlichkeit *p* für verschiedene Längen *L* der Zeitfenster und für verschiedene Abstände *H* zwischen dem Ende des Zeitfensters und dem Ereignis zu betrachten, wie sie in Figur 2 exemplarisch für einen Ereignispatienten (Figur 2a) und einen Kontrollpatienten (Figur 2b) für einen Primärparameter in der *L*-*H*-Ebene, der sog. Beobachtungsebene dargestellt sind. Zudem kann man durch ein Vorzeichen kenntlich machen, ob es sich um einen Auf- oder Abwärtstrend handelt (+*p*, bzw. -*p*). *Lₘᵢₙ* und *Lₘₐₓ* bezeichnen die minimale bzw. die maximale Länge der Zeitfenster, *Hₘᵢₙ* und *Hₘₐₓ* das minimale bzw. maximale Intervall zwischen Vorhersagezeitpunkt und Ereignis. Im schraffierten Bereich (0≤*L*≤2) ist eine Trendberechnung nicht sinnvoll, da für *L*=2 bis auf den Fall identischer Messwerte immer ein Trend vorhanden ist. Im Bereich 3≤*L*≤7 führt eine Trendberechnung nicht zwangsläufig zu stabilen Ergebnissen für das betrachtete Problem. Die Struktur dieser Beobachtungsebene unterscheidet sich für Ereignis- und Kontrollpatienten.

Auf Grund dieser unterschiedlichen Struktur ist in bestimmten Gebieten eine besonders hohe Trennstärke zu erwarten. Form, Länge und Größe dieser Gebiete sind vom jeweiligen Primärparameter *Sⱼ* abhängig. Zur Bestimmung von Form, Lage und Größe dieser Gebiete mit hoher Trennstärke eignet sich z.B. aber nicht ausschließlich die Methode des Fisher-Scores:

Seien für ein *p*(*H*,*L*) in der Beobachtungsebene die Daten *p_{k,H,L}* , (*k*=1, ...,*m*) zweier Klassen (+ und - oder Ereignis und Kontrollklasse) mit der Anzahl *n*₊ und *n*₋ positiver und negativer Klassenelemente gegeben, dann berechnet sich dafür der Fisher-Score zu ${F}_{H , L} = \frac{{\left({\overline{p}}_{H , L}^{+}-{\overline{p}}_{H , L}\right)}^{2} + {\left({\overline{p}}_{H , L}^{-}-{\overline{p}}_{H , L}\right)}^{2}}{\frac{1}{{n}_{+} - 1} {\sum }_{k = 1}^{{n}_{+}} {\left({p}_{k , H , L}^{+}-{\overline{p}}_{H , L}^{+}\right)}^{2} + \frac{1}{{n}_{-} - 1} {\sum }_{k = 1}^{{n}_{-}} {\left({p}_{k , H , L}^{-}-{\overline{p}}_{H , L}^{-}\right)}^{2}}$
mit dem Durchschnitt des ganzen (*p̅*_{*H*,*L*}), positiven (*p̅*_{*H̅*,*L*}) und negativen Datensets (*p̅*_{*H̅*,*L*}) und dem k-ten Wert der positiven $\left({p}_{k , H , L}^{+}\right)$ bzw. negativen Klasse $\left({p}_{k , H , L}^{-}\right) .$

Es ist auch möglich, die Trendwahrscheinlichkeiten mehrerer Primärparameter als kombinierte Größe in die Berechnung der Trennstärken einfließen zu lassen.

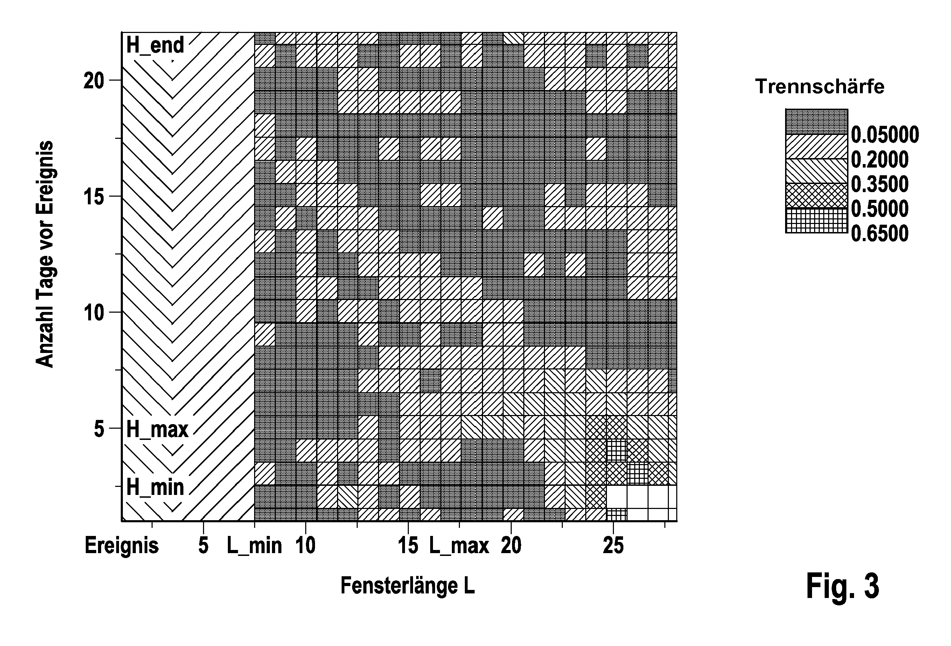

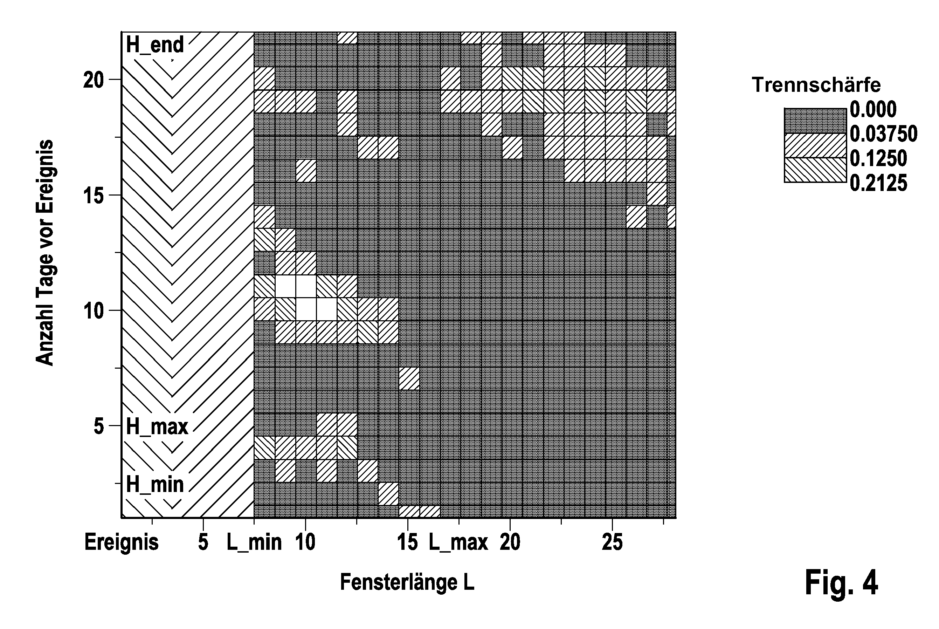

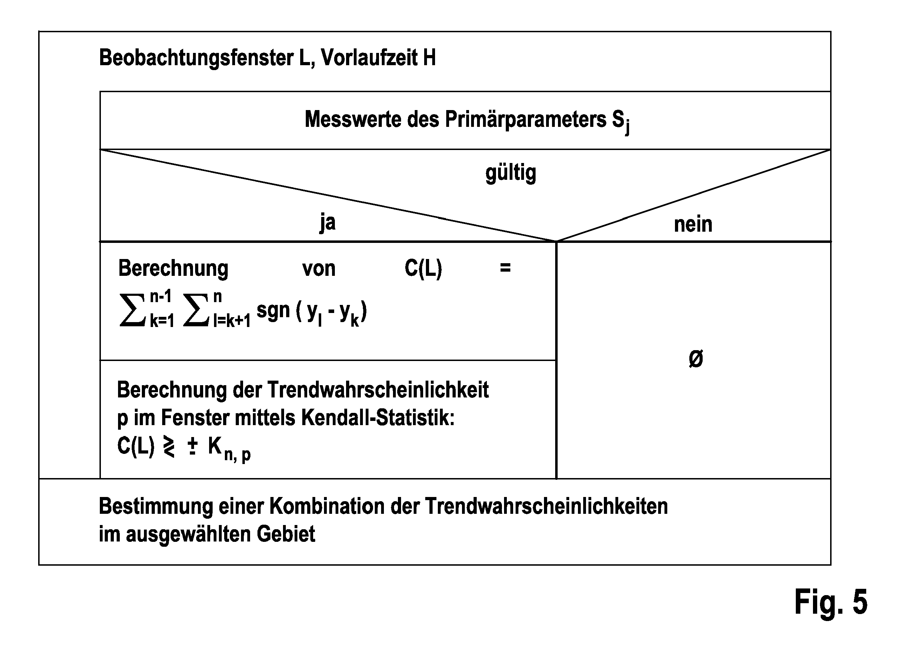

Figur 3 zeigt die Trennschärfe des Primärparameters aus den Figuren 2a und 2b für ein Patientenkollektiv. Die Struktur mit den Gebieten höherer Trennstärke ist hier deutlich erkennbar. Die Güte der Separation zwischen Ereignis- und Kontrollpatienten ist höher bei besonderer Berücksichtigung von Zeitfenstern aus den Gebieten hoher Trennschärfe. Die Berücksichtigung erfolgt durch Gewichtung der Trendwahrscheinlichkeiten aus Figur 2. Diese Gewichte lassen sich z.B. aber nicht ausschließlich durch eine Funktion des Fisher-Scores darstellen, also durch *f*(*F*(*L*,*H*)). Diese Gewichte können für verschiedene Primärparameter verschieden sein. Anschließend werden die gewichteten Wahrscheinlichkeiten z.B. durch Summation kombiniert. Das Gebiet hoher Trennschärfe für einen Primärparameter *Sⱼ* ist nicht notwendigerweise zusammenhängend, wie man z.B. in Figur 4 erkennen kann. Figur 5 zeigt ein Nassi-Shneiderman-Diagramm für die allgemeine Berechnung von Kombinationen der Trendwahrscheinlichkeiten.

Die auf diese Weise erhaltenen Werte sind sog. Merkmale M mit $M = {\sum }_{L , H} p \left(L⁢H\right) f \left(F\left(L⁢H\right)\right)$
und eignen sich somit als Eingangswerte für Klassifikationsverfahren Δ(*M*). Manchmal ist es von Vorteil, *k* nicht zusammenhängende Gebiete als *m* Merkmale in die Klassifikation eingehen zu lassen. Ebenfalls ist es manchmal von Vorteil Auf- und Abwärtstrends als gesonderte Merkmale in die Klassifikation eingehen zu lassen. Dies gilt auch für Merkmale, die aus Kombination mehrerer Primärparameter gebildet werden.

### Optimierungsprozess:

Um die Merkmalsmenge dem Auftreten von Ereignissen zuordnen zu können, ist es erforderlich, ein Klassifikationsverfahren Δ einzusetzen, das selbst wiederum angepasst werden muss. Für diese Optimierung sind für jeden eingesetzten Primärparameter klassifizierte Ereignisdaten und z.B. entsprechende Kontrolldatensätze ohne Ereignis erforderlich. Für eine möglichst hohe Güte des Separationsverfahrens ist die Verwendung von Datensätzen mit gesicherten Ereignissen und unter Umständen eine sorgfältige Auswahl der Kontrolldaten erforderlich.

Es wird nun für jeden Primärparameter *Sⱼ* bzw. für mindestens eine Primärparameterkombination mindestens ein Merkmal M bestimmt. Derartig klassifizierte Merkmale eignen sich für automatisiertes Lernen in Abhängigkeit von der Anzahl von Ereignis- und Kontrolldatensätzen. Hier haben sich Verfahren wie künstliche neuronale Netze, Support Vector Machines, Hidden Markov Modelle oder ähnliche bewährt. Aufgrund der hohen Anzahl von Sätzen freier Parameter während des Trainingsprozesses wird bei diesen Verfahren eine große Anzahl klassifizierter Merkmale benötigt.

Liegen diese Merkmale nicht in der für diese Verfahren benötigten Häufigkeit vor, so ist ein Klassifikationsverfahren zu verwenden, bei dem die Anzahl freier Parameter mit der Anzahl zur Verfügung stehender Trainingsdatensätzen verträglich ist. Im Folgenden soll exemplarisch eine solche Möglichkeit vorgestellt werden:

Zunächst wird aus den erhaltenen Merkmalen ein Merkmalsvektor $\vec{M} = \left({M}_{1}⁢{M}_{2}…{M}_{q}\right)$
gebildet, der dann skalar mit einem Klassifikationsvektor $\vec{α} = \left({α}_{1}⁢{α}_{2}…{α}_{q}\right)$
multipliziert wird. Das Ergebnis dieser skalaren Multiplikation ist eine Maßzahl für das Auftreten eines Ereignisses. Während des Trainingsprozesses wird für Event- bzw. Kontrolldaten der Ausgangswert auf einen fixen Wert 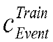 bzw. 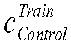 gesetzt, der oberhalb, bzw. unterhalb des Trainingsschwellwertes *c*_{0*,Train*} gelegt wird. Im anschließenden Testverfahren muss ein *c*_{0,*Class*} gefunden werden, damit das Verfahren eine vorgegebene Spezifität und/oder Sensitivität erreicht. Für die Komponenten des Klassifikationsvektors ä können diskrete Werte (z.B, -1, 0, +1 oder ein feineres Gitter) vorgegeben werden, um die Anzahl der Freiheitsgrade weiter einzuschränken. Es kann auch durch systematische Variation der Komponenten von ä ein maximaler Abstand zwischen *cₑᵥₑₙₜ* und *c_{control}* erreicht werden. Die in diesem Beispiel eingesetzte Klassifikationsfunktion hat die Form ${Δ}_{Bsp} = {\sum }_{j = 1}^{q} {α}_{j} ⁢ {M}_{j} .$

Im Allgemeinen besteht die Einschränkung in diesem Beispiel auf einen skalaren Schwellenwert nicht, so dass als Entscheidungsgrenze eine Hyperebene im *Rⁿ* zu verwenden ist.

### Anwendung des Verfahrens:

Nach Abschluss der Optimierung kann das Klassifikationsverfahren zur Vorhersage eines Ereignisses für die Zeitspanne *Hₘᵢₙ* bis *Hₘₐₓ* vom Prädiktionszeitpunkt aus gesehen in der Zukunft liegend eingesetzt werden. Die oben definierten Merkmale M können als Input für ein gewähltes Klassifikationsverfahren Δ dienen. Ein System kann nun über die Zeit überwacht werden, das Verfahren wird nach jedem Eintreffen eines neuen Messwertes oder in größeren Abständen erneut in Gang gesetzt, um eine Vorhersage über Normabweichungen des Systems zu treffen. Wird der im Rahmen des Optimierungsverfahrens bestimmte Schwellenwert *c*_{0,*Class*} über- oder unterschritten, so lassen sich -abhängig vom Schwellenwert- vordefinierte Maßnahmen, wie z.B. eine therapeutische Aktionskette in Gang setzen.

### Umgang mit ungültigen und/oder fehlenden Messwerten:

Vor dem Erzeugen der Merkmale werden die zur Merkmalsberechnung herangezogenen Messwerte der verwendeten Messgrößen einer Gültigkeitsprüfung unterzogen. Einzelne Messwerte können entweder verworfen werden, da sie z.B. nach einer Validitätsprüfung als ungültig markiert werden, weil sie z.B. außerhalb eines vordefinierten Wertebereiches liegen oder sie fehlen, weil sie innerhalb der Übertragungsstrecke auf irgendeine Weise verloren gehen. Das Verfahren kann ungültige und fehlende Messwerte z.B. auf folgende Weise handhaben:
- Beim Test auf Trend nach Mann nach Gleichung (1) werden derartige Messwerte ignoriert, indem sich in diesem Fall die effektive Fensterlänge um die Anzahl dieser Messwerte reduziert.
- Ungültige und/oder invalide Messwerte werden durch entsprechende Werte aus einem Interpolationsverfahren ersetzt.
- Nicht berechenbare Wahrscheinlichkeiten *p* auf Grund von ungültigen und/oder invaliden Messwerten werden durch einen Mittelwert (Gebietsmittel, Mittel der Beobachtungsebene, Gruppenmittel oder andere) ersetzt.
- Eine andere Möglichkeit zur Handhabung von missing values wird verwendet.

Nachstehend wird die Erfindung an einem Ausführungsbeispiel näher erläutert.

Als Ausführungsbeispiel dient ein Gerät zur Vorhersage einer sog. Dekompensation bei Patienten, die an chronischem Herzversagen bzw. Stauungsinsuffizienz des Herzens (CHF, chronic/congestive heart failure) leiden. Die Erfindung ist jedoch nicht auf den medizinischen Bereich eingeschränkt. Vielmehr ist sie auch nutzbar, um Ereignisse in anderen komplexen Systemen, wie etwa meteorologischen, geophysikalischen Systemen oder auch in volks- oder betriebswirtschaftlichen Systemen.

Kennzeichnend für diese Patienten ist, dass trotz eines generell chronischen Verlaufs der Erkrankung zeitweise Phasen eines akuten und kurzfristig lebensbedrohlichen Zustandes eintreten, die eine Hospitalisierung erfordern. Da die Hospitalisierung mit hohen Kosten und das Eintreten einer Dekompensation mit höherer Mortalität und Morbidität verbunden ist, bemüht man sich um deren Vermeidung durch einen frühzeitigen therapeutischen Eingriff. Voraussetzung dafür ist das frühzeitige Erkennen einer möglichen Dekompensation.

Eine Reihe von CHF-Patienten bietet Indikationen zur Implantation eines elektrisch aktiven Implantates. Dies ist meist ein CRT-Schrittmacher (CRT = Cardiac Resynchronization Therapy), um durch eine Wiederherstellung der Synchronisation der Herzkammern die Ursachen einer Pumpschwäche des Herzens direkt zu bekämpfen. Zur Absicherung gegen den plötzlichen Herztod kann bei myokardialer Insuffizienz auch ein automatischer Defibrillator implantiert werden. Bei entsprechenden technischen Einrichtungen ist es möglich, über die Sonden und Sensoren dieser Implantate verschiedene Messgrößen physiologischer oder technischer Natur zu bestimmen. Diese Messgrößen lassen sich entweder direkt in dem Implantat verarbeiten oder z.B. in einem externen Rechensystem auswerten, indem die Messgrößen vom Implantat über eine Telemetriestrecke an den behandelnden Arzt übertragen werden. Dabei kommen auch andere Implantate oder externe Gerätetypen in Frage.

Da somit ein Anteil von CHF-Patienten derzeit ein elektrisch aktives Implantat (CRT-Schrittmacher, ICD = Implantable Cardioverter Defibrillator) erhält, bietet es sich an, die Früherkennung einer CHF-Dekompensation im Rahmen einer Homemonitoring-Lösung anzustreben. Die hierzu ausgeführte Methodik ist nicht zwingend an Implantate gebunden.

Die Sensoren umfassen je nach Ausführung Elektroden zur Erfassung des intrakardialen Elektrogrammes, zur Bestimmung von Impedanzen, des Druckes und weitere Messgrößen. Die Messgrößen ihrerseits lassen sich einer optionalen Zwischenverarbeitung unterziehen oder direkt einer Analyse im Hinblick auf die Diagnose eines Patienten zuführen. Die Erzielung von belastbaren diagnostischen Aussagen ist jedoch mit einer Reihe von Schwierigkeiten behaftet.

Die Grunderkrankung CHF beschreibt einen komplexen pathophysiologischen Prozess, dem vielfältige Ursachen zu Grunde liegen. Unterschieden werden dabei z. B. ischämische, nicht-ischämische, dilatative, toxische oder idiopathische Auslöser von CHF. Faktoren, wie z. B. mangelhafte Patientencompliance bei Medikamenteneinnahme oder Einhaltung der Diät, akute Atemwegserkrankungen, Infektionen oder andere Komorbiditäten erschweren zusätzlich die Beschreibung dieser Grunderkrankung.

Wegen der Komplexität der Grunderkrankung und dem Dekompensationsverlauf sind die gemessenen Patientensignale heterogen vor Auftreten der zu beobachtenden Ereignisse. Dies bedeutet, dass bei verschiedenen Patienten auch ein unterschiedlicher Signalverlauf zu erwarten ist, und ebenso wird auch der Verlauf zweier verschiedener Dekompensationsereignisse bei ein und demselben Patienten unterschiedlich ausfallen. In beiden Fällen können zudem Lücken in der Messreihe auftreten. Ursache hierfür kann das Verwerfen von Messwerten nach einer Gültigkeitsprüfung oder der Verlust von Messwerten auf der Telemetriestrecke sein.

Die Heterogenität im Signalverlauf vor Ereignissen erstreckt sich auf mehrere verschiedene Aspekte:
- Erstens treten Unterschiede dahin gehend auf, in welcher Messgröße sich Änderungen widerspiegeln. So kann es sein, dass erste Ansätze für eine Dekompensation sich zuerst im Niveau der mittleren Herzrate zeigen, Impedanzänderungen erst im Rahmen der eigentlichen Dekompensation auftreten, es kann jedoch auch umgekehrt sein.
- Zweitens ist die Reaktion des Patienten auf eine beginnende Dekompensation in gewissem Maße verhaltensabhängig. Dies bedeutet, dass einzelne Messgrößen bei einer Gruppe von Patienten ansteigen, während sie bei einer anderen Gruppe abfallen, wenn die eine z.B. aufgrund von Nervosität aktiver wird, die andere aus Ängstlichkeit sich eher zurückgezogen verhält.
- Drittens wird sich die Progression des Krankheitsverlaufes vor einer Dekompensation im Allgemeinen hinsichtlich des Zeitverlaufes unterscheiden, der von der individuellen Konstitution, dem Verhalten oder anderen Faktoren abhängt. Insbesondere können weitere nicht bekannte, nicht messbare, nicht kontrollierbare und individuelle Faktoren in der Grundgesamtheit der CHF-Patienten auftreten.

Es ist erforderlich, eine kontinuierliche Überwachung zu gewährleisten, d. h. nach Möglichkeit mindestens einmal am Tag Messungen durchzuführen. Zudem sind Belastungen für den Patienten und Kosten zu vermeiden, die entstehen können, wenn zu häufig Fehlalarme auftreten und daraus folgend der Patient zu häufig kontaktiert und/oder hospitalisiert wird oder sogar überflüssige diagnostische und/oder inadäquate therapeutische Eingriffe vorgenommen werden. Die Verfahren müssen daher auch eine hohe Spezifität, d. h. eine sehr niedrige Fehlalarmquote aufweisen und daher eine zuverlässige unterstützende Entscheidungsgrundlage für den Arzt bereitstellen. Zusätzlich soll mit einer hohen Sensitivität die Dekompensation eines möglichst breiten Patientenkollektivs rechtzeitig erkannt werden.

Zur Gewährleistung einer hinreichenden statistischen Güte ist es daher erforderlich, ein Verfahren zu verwenden, das robust gegenüber den oben genannten Heterogenitäten ist. Dabei kommt aufgrund der Natur der Erkrankung hinzu, dass ein konkretes Verlaufsmuster einer Dekompensation für den jeweiligen Patienten nicht a-priori bekannt sein kann.

Im Folgenden soll durch eine exemplarische Wahl der Parameterwerte die Handhabung des Verfahrens veranschaulicht werden.
Beobachtungsfensters: *Lₘᵢₙ*=8, *Lₘₐₓ*=28
Vorlaufzeit der Prädiktion: *Hₘᵢₙ*=1 , *Hₘₐₓ*=3, *H_{end}*=20
Anzahl Primärparameter: 8 $Gewichtungsfunktion : f \left(F\left(L⁢H\right)\right) = \left\{\begin{matrix}1 für F \left(l⁢H\right) \geq {F}_{O} \\ 0 sonst\end{matrix}\right\}$
Anzahl Merkmale: q=10 (auf Grund zweier Primärparameter mit je zwei disjunkten Gebieten)
geforderte Trennschärfen: *F_{0,1}* = *F_{0,2}*=...=*F_{0,10}*= 0.5

Die Angabe der einzelnen Gebietsgrenzen ist stark geräteabhängig und trägt an dieser Stelle nicht zum Verständnis bei.

Die Primärparameter können aus folgender Menge gewählt werden:
- Alle Messgrößen, die die Herzrate des Patienten beinhalten, wie z.B. die Herzrate über einen vordefinierten Zeitraum, die Herzrate während einer definierten Ruhephase, die Variabilität der Herzrate und ähnliche
- Alle Größen, die Impedanzen im Patienten bestimmen, sei es durch intrakardiale (bipolare und multipolare), intrathorakale Messungen oder durch Bestimmung mit externen Sensoren
- Alle Messgrößen, die die Aktivität eines Patienten auf irgendeine Weise bestimmen
- Alle Messgrößen, in denen der Anteil links- oder rechtsventrikulärer stimulierter, wahrgenommener oder anderer Events einfließt
- Alle Messgrößen, die implantatabhängige Signale aufzeichnen
- Alle Messgrößen, die Extrasystolen unabhängig ihres Ursprungsorts bestimmen
- Alle Messgrößen, die die Hämodynamik eines Patienten oder sonstige Elastizitäten, Drücke, Volumina oder Abstände erfassen
- Alle Messgrößen, die außerhalb eines Implantats bestimmt werden, wie Größen, die durch aus Funk angebundenen Sensoren gewonnen werden oder Größen von Geräten, die Daten außerhalb des Körpers aufzeichnen und diese telemetrisch der Auswertungseinheit übermitteln
- Alle biomedizinischen Messgrößen wie Stimulationsschwellen, Elektrodenkonfigurationen, Sensorverstärkungen oder Versatzwerte
- Alle Messgrößen wie Blutzuckerspiegel, andere Biomarker oder ähnliche Größen
- Alle Messgrößen, die biometrische Informationen über den Patienten bestimmen
- Alle Messgrößen, die zusätzliche Informationen über eine Medikamentierung aufzeichnen
- Alle Messgrößen die aus elektrophysiologischen oder biochemischen Verfahren bestimmt werden
- Alle Messgrößen, die Signale aus bildgebenden, akustischen oder mechanischen Verfahren erfassen
- Alle oben genannten Messgrößen, die zuvor normiert und/oder skaliert wurden
- Mögliche Kombinationen mehrerer der oben genannten Messgrößen

Dadurch ergibt sich für oben genannte Parameterwerte, z.B. folgende Klassifikationsfunktion: ${Δ}_{Bsp} = {\sum }_{j = 1}^{10} {α}_{j} ⁢ {M}_{j} .$

Die Werte der α*ⱼ* wurden nach Vorgabe diskreter Werte im Trainingsprozess wie weiter oben beschrieben optimiert. Eine genauere Spezifikation der Primärparameter ist für das Verständnis des Ausführungsbeispiels nicht nötig.

Die Erfindung beschränkt sich in ihrer Ausführungsform nicht auf das vorstehend angegebene bevorzugte Ausführungsbeispiel. Vielmehr ist eine Anzahl von Varianten denkbar, die von der erfindungsgemäßen Anordnung, dem erfindungsgemäßen Verfahren, dem erfindungsgemäßen Computerprogramm und dem erfindungsgemäßen entsprechenden computerlesbaren Speichermedium auch bei grundsätzlich anders gearteten Ausführungen Gebrauch macht.

## Patentansprüche

1. Verfahren zur Vorhersage mindestens eines Systemereignisses, wobei das Verfahren zumindest folgende Schritte umfasst:
Erfassung von Messwerten sⱼ mindestens eines Primärparameters Sⱼ innerhalb mindestens eines Zeitfensters vorgebbarer Länge,
für zumindest einen Teil der erfassten Primärparameter Sⱼ Ermittlung einer Trendwahrscheinlichkeit pⱼ für eine vorgebbare Anzahl von Zeitfenstern,
für zumindest einen Teil der erfassten Primärparameter Sⱼ Ermittlung eines Wertes für die Relevanz der Zeitfenster für die Vorhersage des mindestens einen Systemereignisses,
für zumindest einen Teil der erfassten Primärparameter Sⱼ und für zumindest einen Teil der Zeitfenster Konstruktion mindestens eines Merkmals M zur Beschreibung des Systems durch Auswertung der Trendwahrscheinlichkeit(en) pⱼ und des Wertes für die Relevanz der Zeitfenster,
Klassifikation des mindestens einen Merkmals M und
Signalisierung eines Systemereignisses und/oder Einleitung systemereignisbezogener Maßnahmen in Abhängigkeit der Ergebnisse der Klassifikation.

2. Verfahren nach Anspruch 1,
**dadurch gekennzeichnet, dass**
die Ermittlung des Wertes für die Relevanz der Zeitfenster die Ermittlung einer Trennschärfe des Zeitfensters umfasst.

3. Verfahren nach Anspruch 1 oder 2,
**dadurch gekennzeichnet, dass**
die Auswertung der Trendwahrscheinlichkeiten pⱼ eine Gewichtung der Trendwahrscheinlichkeiten pⱼ umfasst.

4. Verfahren nach einem der voranstehenden Ansprüche,
**dadurch gekennzeichnet, dass**
die Trendwahrscheinlichkeiten pⱼ für Zeitfenster mit einer Länge L, Lₘᵢₙ ≤ L ≤ Lₘₐₓ, und für Vorhersagezeitpunkte H, Hₘᵢₙ ≤ H ≤ Hₘₐₓ ermittelt werden.

5. Verfahren nach einem der voranstehenden Ansprüche,
**dadurch gekennzeichnet, dass**
ein für die Klassifikation genutztes Klassifikationsverfahren unter Verwendung von klassifizierten Ereignisdaten und/oder von Kontrolldaten optimiert wird.

6. Verfahren nach einem der voranstehenden Ansprüche,
**dadurch gekennzeichnet, dass**
für einen Primärparameter Sⱼ mehrere Merkmale konstruiert werden.

7. Verfahren nach einem der voranstehenden Ansprüche,
**dadurch gekennzeichnet, dass**
bei der Konstruktion des mindestens einen Merkmals M auf- und absteigende Trends gemeinsam oder getrennt berücksichtigt werden.

8. Verfahren nach einem der voranstehenden Ansprüche,
**dadurch gekennzeichnet, dass**
bei der Konstruktion des mindestens einen Merkmals M die Trendstärke berücksichtigt wird.

9. Verfahren nach einem der voranstehenden Ansprüche,
**dadurch gekennzeichnet, dass**
die Messwerte sⱼ von einem aktiven oder passiven Implantat geliefert werden.

10. Verfahren nach einem der voranstehenden Ansprüche,
**dadurch gekennzeichnet, dass**
es sich bei dem System um
- ein medizinisches,
- ein meteorologisches,
- ein geophysikalisches oder
- ein volks- oder betriebswirtschaftliches
System handelt.

11. Anordnung mit mindestens einem Chip und/oder Prozessor, wobei die Anordnung derart eingerichtet ist, dass ein Verfahren zur Vorhersage mindestens eines Systemereignisses gemäß einem der Ansprüche 1 bis 10 ausführbar ist.

12. Anordnung nach Anspruch 11,
**dadurch gekennzeichnet, dass**
die Anordnung ein aktives oder passives Implantat zur Erfassung der Messwerte sⱼ umfasst.

13. Computerprogramm, das es einer Datenverarbeitungseinrichtung ermöglicht, nachdem es in Speichermittel der Datenverarbeitungseinrichtung geladen worden ist, ein Verfahren zur Vorhersage mindestens eines Systemereignisses gemäß einem der Ansprüche 1 bis 10 durchzuführen.

14. Computerlesbares Speichermedium, auf dem ein Programm gespeichert ist, das es einer Datenverarbeitungseinrichtung ermöglicht, nachdem es in Speichermittel der Datenverarbeitungseinrichtung geladen worden ist, ein Verfahren zur Vorhersage mindestens eines Systemereignisses gemäß einem der Ansprüche 1 bis 10 durchzuführen.

15. Verfahren, bei dem ein Computerprogramm nach Anspruch 13 aus einem elektronischen Datennetz, wie beispielsweise aus dem Internet, auf eine an das Datennetz angeschlossene Datenverarbeitungseinrichtung heruntergeladen wird.
